Europäisches Patentamt

**European Patent Office**

**Office européen des brevets**

(11) Publication number: **0 252 214**
**A1**

(12) **EUROPEAN PATENT APPLICATION**

(21) Application number: **87103311.4**

(22) Date of filing: **08.03.87**

(51) Int. Cl.³: **A 61 M 31/00**

(30) Priority: **01.04.86 IT 421886 U**
**05.11.86 IT 425686 U**

(43) Date of publication of application:
**13.01.88 Bulletin 88/2**

(84) Designated Contracting States:
**AT BE CH DE ES FR GB GR IT LI LU NL SE**

(71) Applicant: **SERIOPLAST S.R.L.**
**Via P. Elzi**
**I-24050 Grassobbio (Bergamo)(IT)**

㉟{72} Inventor: **Innocenti, Dario**
**Via Molino Vecchio 5**
**I-24068 Seriate (Bergamo)(IT)**

㉟{74} Representative: **Giambrocono, Alfonso, Dr. Ing. et al,**
**Ing. A. Giambrocono & C. S.r.l. Via Rosolino Pilo 19/B**
**I-20129 Milano(IT)**

(54) **Syringe for the rectal insertion of suppositories.**

㉠ The syringe for the rectal insertion of suppositories (2A) is characterized by a plunger (8) mobile within a hollow casing (5R) having an expulsion end (1A) which is elastically yieldable but possesses sufficient rigidity for its possible insertion into the anus and is of a shape suitable for retaining in its interior the suppository (2A) until the plunger (8) operates, and further which is adapted for fixing to the hollow casing (5R).

Fig. 1

Croydon Printing Company Ltd.

Description

This invention applies to the medical instrument field, and relates to a syringe which allows rectal insertion of suppositories without hand contact.

Suppository insertion is known to involve contact between the fingers and the mucous membranes of the anal sphincter. This causes indesiderable psychological disturbance to some people because of the need for contact with parts theoretically in contact with the feces. These parts are precisely those most subject to the transmission of germ and virus infections, so that if this procedure is carried out on other people, rubber gloves have to be used, to be then disposed of.

An object of the present invention is to provide an instrument which enables suppositories to be inserted without bringing the hands into contact with the anus.

A further object is to provide an instrument which enables the suppository to be inserted without irritating the sphincter.

A further object is to provide an instrument able to operate with suppositories of different diameter.

A further object is to provide an instrument which exerts no extrusion action on the suppositories during insertion, which would cause suppository soiling and volume reduction.

A further object is to provide an instrument of limited length which is light and easy to use.

A further object is to provide an instrument in which its manually handled materials can be of different hardness from its materials which make contact with mucous membranes.

A further object of the invention is to provide a syringe which makes possible to insert safely suppositories beyond the sphinteral rejection point of the sphincter.

These and further objects which will be more apparent to experts of the art from the description and claims given hereinafter are attained by a syringe for the rectal insertion of suppositories, characterized by a plunger mobile within a hollow casing having a lateral aperture for loading the suppository and an expulsion end which is elastically yieldable but possesses sufficient rigidity for its possible insertion into the anus and is of a shape suitable for retaining in its interior the suppository until the plunger operates, and further which is adapted for fixing to the hollow casing.

The invention is illustrated by way of non-limiting example on the accompanying drawings, in which:

Figure 1 is a perspective view of a syringe with the side loading;

Figure 2 is a partial axial section showing the system for fixing the expulsion end to the hollow casing in the embodiment of figure 1.

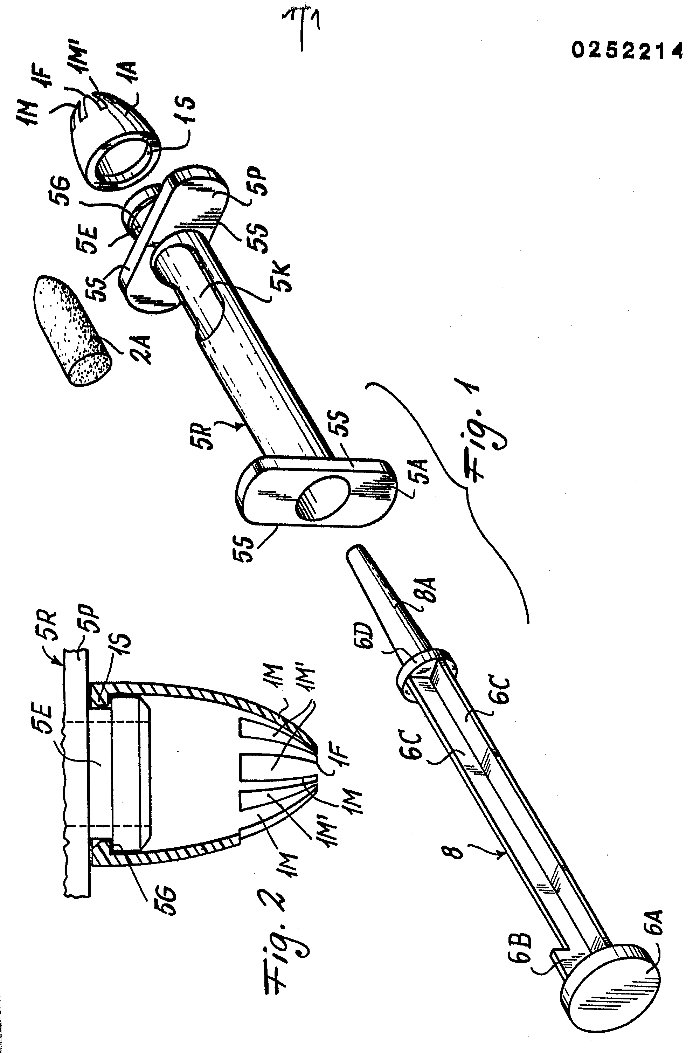

The syringe of figure 1 comprises a hollow casing 5R of plastics material and a piston or plunger 8 also of plastics material which is slidably mounted in said casing. The casing 5R is provided with a removable ogival end portion 1A. The ogival end portion 1A is fixed to the casing 5R by snap-engagement over a step 5G, on which an edge 1S on the inside of said ogival end 1A rests.

A suppository 2A is contained partly in the ogival end portion 1A and partly in the end 5E of the hollow casing 5R, and is inserted through a lateral aperture 5K of the latter.

The ogival end portion 1A is formed of plastics material of easy elastic deformability, i.e. of plastics material which is more elastically deformable than the plastics material used for the hollow casing 5R and the piston 8.

The ogival end portion 1A is provided at its free end with a central hole 1F a series of radial cuts or separation channels 1M' defining there between elastically easy deformable lobes 1M ending the central hole 1F, the diameter of which is smaller than the maximum diameter of the suppository 2A. The ogival end portion 1A being thus provided with elastically openable petals (lobes 1M) has the advantageous property of being able to house and retain suppositories of various dimensions while using constant dimension syringes. This enables the syringe to be standardised as a single model, with considerable economical advantages related to the smaller cost of its moulds.

The hollow casing 5R is provided with two spaced part ledges 5A which are partially round with parallel flat portions 5S in shape. As the usual orientation of a another persons hand using the syringe would be such that the ledge 5A is positioned vertically, the other ledge 5P must have its major axis perpendicular to this so that it becomes disposed transversely to the vertical line separating the buttocks.

The piston 8 has an end ledge 6A, a series of spaced apart longitudinal radially extending fins 6C having enlargements 6B at the end ledge 6A thus creating a spacer between the two ledges 6A, 5A. The fins 6C terminates on a disk shaped portion 6D and ends with a conically tapered point 8A which exerts the thrust on the suppository. The total length of the plunger 8 is such that at least part of the point 8A projects beyond the end hole 1F (thus penetrating adeguately deep into the anus to avoid the repulsive force on the suppository exerted by the sphinctes) when the enlargements 6B touch the ledge 5A.

Claims:

1. A syringe for the rectal insertion of suppositories, characterized by a plunger (8) mobile in a hollow casing (5R) having a lateral aperture for loading the suppository (2A) and an elastically yieldable expulsion end (1A) of such a shape as to retain the suppository (2A) until the plunger (8) operates.

2. A syringe as claimed in claim 1, characterized in that the expulsion end (1A) comprises strip portions (1M) which elastically diverge under the thrust exerted by a plunger (8) on the suppository (2A) to enable this latter to emerge.

3. A syringe as claimed in claim 1 or claims 1 and 2, characterized by an expulsion end (1A) removably fitted to the hollow casing (5R).

4. A syringe as claimed in the preceding claims, characterized by an expulsion end (1A) which in its function of containing the suppository (2A) is assisted by the end (5E) of the hollow casing (5R).

5. A syringe as claimed in the preceding claims, characterized in that the hollow casing (5R) comprises a first elongated ledge (5A) which is orientated perpendicular to a second ledge (5P) of said hollow casing (5R), which is designed to rest against the patient.

6. A syringe as claimed in the preceding claims, characterized in that the plunger (8) is of cross-shaped cross-section and comprises transverse enlargements (6B) for maintaining a distance between the free end (6A) of the plunger (8) and a first elongated ledge (5A) of the hollow casing (5R).

7. A syringe as claimed in one or more of the preceding claims, characterized in that the plunger (8) has a frustoconical end (8A).

8. A syringe as claimed in one or more of the preceding claims, characterized in that the plunger (8) is longer than the hollow casing (5R) so that on termination of the expulsion stroke for the suppository (2A) said plunger (8) partly lies beyond the expulsion end (1A).

Fig. 1

Fig. 2

| | DOCUMENTS CONSIDERED TO BE RELEVANT | | EP 87103311.4 |
|---|---|---|---|

| Category | Citation of document with indication, where appropriate, of relevant passages | Relevant to claim | CLASSIFICATION OF THE APPLICATION (Int Cl 4) |
|---|---|---|---|
| Y | US - A - 2 754 822 (M.EMELOCK) | 1,2,7,8 | A 61 M 31/00 |
| A | * Totality * | 5 | |
| Y | US - A - 2 647 512 (J.R. JOHNSON) | 1,2,7,8 | |
| A | * Totality; especially column 1, lines 49-52 * | 5 | |
| A | US - A - 1 538 678 (J.S. BLINN) | 1-3,7,8 | |
| | * Totality; especially fig. 1-3; page 1, lines 44-46 * | | |
| A | FR - A - 1 566 319 (G. REVELLI) | 1-3 | |
| | * Totality; especially fig. 1; page 2, paragraph 7 * | | |
| A | GB - A - 1 333 095 (KIMBERLEY-CLARK) | 1,2,7,8 | **TECHNICAL FIELDS SEARCHED (Int Cl 4)** A 61 M 31/00 |
| | * Totality * | | |
| A | DE - A1 - 3 031 408 (F. MÄCHTLE) | 1,5,6 | |
| | * Totality; especially fig. 1,3; page 10, lines 19-32; page 11, lines 20-27; page 13, lines 9-14 * | | |

The present search report has been drawn up for all claims

| Place of search | Date of completion of the search | Examiner |
|---|---|---|
| VIENNA | 22-09-1987 | LUDWIG |